Europäisches Patentamt

(19) European Patent Office

Office européen des brevets

(11) **EP 1 111 535 A1**

(12) **DEMANDE DE BREVET EUROPEEN**

(43) Date de publication:
**27.06.2001 Bulletin 2001/26**

(51) Int Cl.⁷: **G06F 19/00**

(21) Numéro de dépôt: **00403459.1**

(22) Date de dépôt: **11.12.2000**

(84) Etats contractants désignés:
**AT BE CH CY DE DK ES FI FR GB GR IE IT LI LU MC NL PT SE TR**
Etats d'extension désignés:
**AL LT LV MK RO SI**

(30) Priorité: **21.12.1999  FR 9916171**

(71) Demandeur: **L'OREAL**
**75008 Paris (FR)**

(72) Inventeurs:
• **Loussouarn, Geneviève**
  **92110 Clichy (FR)**
• **Huber, Catherine, UFR Biomédicale**
  **75270 Paris (FR)**
• **Mourad, Mamoud, UFR Biomédicale**
  **75270 Paris (FR)**

(74) Mandataire: **Casalonga, Axel**
**BUREAU D.A. CASALONGA - JOSSE**
**Morassistrasse 8**
**80469 München (DE)**

(54) **Système et procédé d'analyse et de simulation prévisionnelle de l'évolution d'une zone pileuse, et plus particulièrement du cuire chevelu humain**

(57)   Système et simulation de l'évolution d'une zone pileuse du cuir chevelu d'un sujet au cours du temps, comprenant un moyen d'observation de ladite zone pileuse apte à fournir en sortie des données d'observation, un premier moyen de traitement capable d'effectuer à partir des données d'observation et de données externes, un classement des cheveux de ladite zone, un second moyen de traitement capable d'effectuer une simulation de l'évolution des cheveux en fonction des données issues du premier moyen de traitement, et un moyen de visualisation des données issues du second moyen de traitement.

EP 1 111 535 A1

**Description**

La présente invention concerne le domaine de la cosmétique et plus particulièrement l'évolution de la chevelure humaine au cours de la vie.

Le phénomène de croissance et de chute des cheveux de l'espèce humaine, plus particulièrement du sexe masculin, est complexe et diffère des espèces animales par au moins deux critères :

- Rares sont les espèces animales qui, avec l'âge, perdent progressivement leur fourrure;
- Les espèces animales peuvent connaître des mues saisonnières en raison du fait que les cycles des poils sont synchronisés, c'est-à-dire que les poils poussent ou chutent tous en même temps.

Chez l'homme, les cheveux sont générés par les follicules pileux implantés dans le cuir chevelu. On dit qu'une chevelure en bonne santé comporte entre 100 000 et 150 000 cheveux, et chaque cheveu au sein de cette chevelure possède un cycle qui lui est propre.

Ce cycle de vie se décompose en trois phases physiologiques généralement successives :

- une phase de croissance du cheveu, appelée Anagène (A) qui peut durer de quelques semaines à 10 ans,
- une phase transitoire d'involution du follicule et d'arrêt de croissance du cheveu avec dégénérescence de la racine, dite Catagène, de l'ordre de quelques semaines,
- une phase d'élimination du cheveu avec remontée de la racine vers la surface, dite Télogène (T), durant environ 1 à 5 mois.

A l'issue de cette dernière phase, le cheveu disparaît donc du cuir chevelu et cette disparition peut s'étendre de quelques jours à quelques mois avant que le follicule ne se réactive pour donner un nouveau cheveu en phase anagène de croissance.

Après un certain nombre de cycles, le follicule arrête définitivement sa production et le cheveu peut être considéré comme Mort (M).

Compte tenu du rapport des durées des différentes phases du cycle de vie du cheveu, dans la pratique, la phase Catagène la plus courte est rarement observée; c'est la raison pour laquelle l'homme de l'art comptabilise de façon privilégiée les phases Anagène (A), Télogène (T) et Disparue (D).

Le phénomène de vieillissement, au cours de la vie, conduit sur des dizaines d'années à un raccourcissement des phases de croissance (A) et par conséquent à une augmentation de la proportion de cheveux en phase de chute (T). Ce phénomène peut s'accélérer dans le cas de l'alopécie qui touche plus particulièrement les hommes mais aussi les femmes, et qui conduit dans son ultime stade à la calvitie, au dégarnissement total de la chevelure.

Les phases du cycle du cheveu se déroulant sur des durées très longues, notamment la phase de croissance (A), il s'ensuit que l'observation de l'état des cheveux d'une zone du cuir chevelu à un instant précis ne constitue qu'une mesure instantanée, non prédictive de l'évolution future.

De manière concrète, l'état actuel des techniques ne permet pas par l'observation et la quantification de l'état des cheveux chez une jeune personne de 25 ans, de prédire et/ou d'illustrer l'état de sa chevelure quand celle-ci aura l'âge de 60 ans.

La présente invention a pour but de simuler l'évolution chronologique d'une chevelure à partir d'un nombre de mesures le plus réduit possible.

Le système, selon l'invention, est destiné à la simulation de l'évolution d'une zone du cuir chevelu d'un sujet au cours du temps, et dont l'extension à l'ensemble du cuir chevelu permet d'illustrer l'évolution globale de la chevelure.

Le système comprend un moyen d'observation de ladite zone pileuse apte à fournir en sortie des données numériques d'observation, un premier moyen de traitement de données numériques capable d'effectuer à partir des données d'observation et de données externes un classement de parties élémentaires de ladite zone, un second moyen de traitement de données numériques capable d'effectuer une simulation d'évolution de ladite zone pileuse en fonction des données issues du premier moyen de traitement de données numériques et un moyen de visualisation des données issues du second moyen de traitement de données numériques. Les données en sortie du premier moyen de traitement comprennent au moins un classement suivant le diamètre des cheveux.

Avantageusement, les données en sortie du premier moyen de traitement comprennent la répartition des cheveux suivant les phases Anagène, Télogène ou Disparue, dans laquelle ils se trouvent, en fonction de leurs diamètres. On peut classer les cheveux en cinq plages de diamètres pour les phases Anagène et Télogène.

Dans un mode de réalisation de l'invention, les données externes comprennent au moins l'âge du sujet.

Avantageusement, le second moyen de traitement est capable de calculer et de prédire la proportion A de cheveux en phase anagène.

Dans un mode de réalisation de l'invention, le second moyen de traitement est capable de calculer et de prédire la densité surfacique de cheveux, la proportion T de cheveux en phase Télogène, la proportion D de cheveux Disparus, et la vitesse de pousse individuelle des cheveux.

Le système peut comprendre un moyen pour effectuer un troisième traitement de simulation de l'évolution de l'ensemble de la chevelure du sujet à partir des données issues du second traitement.

Dans un mode de réalisation de l'invention, le système comprend un moyen pour associer aux données issues du troisième traitement, des données de simulation de l'évolution du visage.

Dans un mode de réalisation de l'invention, le second moyen de traitement comprend un moyen pour estimer le nombre de cycles $n_c$ effectués par un cheveu observé à partir au moins de l'âge du sujet, et pour le comparer à un nombre de cycles maximal prédéterminé Nk, un cycle étant défini par le passage successif dans les trois phases Anagène, Télogène et Disparue.

L'invention a également pour objet un procédé de simulation de l'évolution d'une zone pileuse du cuir chevelu d'un sujet au cours du temps, dans lequel:

- on observe ladite zone pileuse pour fournir des données d'observation
- on effectue un premier traitement des données d'observation pour classer des cheveux de ladite zone pileuse à partir des données d'observation et de données externes,
- on effectue un second traitement pour effectuer une simulation de l'évolution temporelle desdits cheveux en fonction des données issues du premier moyen de traitement, et
- on visualise des données issues du second traitement.

Les données en sortie du premier moyen de traitement comprennent au moins un classement suivant le diamètre des cheveux.

On peut envisager de classer les cheveux par touffes et de calculer un nombre moyen de cheveux par touffes. On peut affecter un numéro à chaque cheveu et à chaque touffe.

Dans un mode de réalisation de l'invention, on effectue au moins une observation, chaque observation étant précédée par une étape de rasage de ladite zone pileuse, l'étape de rasage étant séparée de l'observation correspondante par une première durée donnée.

Dans un mode de réalisation de l'invention, on effectue au moins deux observations séparées par une deuxième durée donnée.

Avantageusement, à partir des données d'observation, on calcule la couverture capillaire en fonction du nombre, du diamètre et de la longueur ou de la vitesse de pousse des cheveux.

Avantageusement, on effectue un troisième traitement pour effectuer une simulation de l'évolution de l'ensemble de la chevelure du sujet à partir des données issues du second traitement et on visualise les données issues du troisième traitement. La visualisation desdites données issues du troisième traitement peut s'effectuer en projection à plat, par exemple en une projection du type utilisé, en cartographie, notamment une projection conique de Lambert. On peut associer aux données issues du troisième traitement, des données de simulation de l'évolution du visage et visualiser les données associées.

Dans un mode de réalisation de l'invention, on simule l'évolution d'une zone pileuse ayant fait l'objet d'une intervention chirurgicale, esthétique ou médicale.

Les données d'observation peuvent être issues de tout moyen adéquat de récolte d'images photographiques et vidéographiques, analogiques ou numériques, ou tout autre moyen d'imagerie obtenu par voie non invasive, respectueuse de la physiologie et ne nécessitant pas de préparation particulière du cuir chevelu.

Dans un mode de réalisation de l'invention, on affecte à un cheveu une durée totale de maintien dans la phase dans laquelle il se trouve, on effectue un tirage aléatoire de la durée écoulée dans ladite phase, on calcule la durée restante dans ladite phase égale à la différence entre la durée totale de maintien et la durée écoulée. L'affectation de la durée totale de maintien peut être effectuée selon une loi composée de Cox et peut tenir compte du diamètre du cheveu, du taux de cheveux en phase Anagène, de la densité de cheveux, du nombre moyen de cheveux par touffes, du numéro de cycle, etc.

En d'autres termes, le système de simulation associe l'établissement de données initiales d'observation, à partir d'une zone de faible surface, du nombre de cheveux, des proportions de ceux-ci dans chaque phase, l'âge du sujet, et la modélisation mathématique de ces derniers qui permet d'étendre à court, moyen ou très long terme l'évolution de chaque paramètre. L'état de la chevelure peut être ainsi simulé sur différentes durées comprises entre 3 mois et 100 ans.

La présente invention sera mieux comprise à l'étude de la description détaillée de quelques modes de réalisation pris à titre d'exemples nullement limitatifs et illustrés par le dessin annexé.

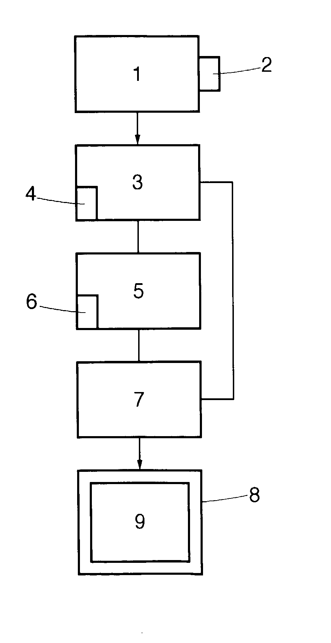

- La figure 1 est une vue schématique du système de simulation conforme à l'invention.

**EP 1 111 535 A1**

Comme on peut le voir sur cette figure, le système de simulation conforme à l'invention comprend une caméra 1, par exemple de type CCD, équipée d'un objectif 2, destinée à l'observation d'une zone pileuse déterminée, par exemple une zone de 1 cm². Le système comprend également un moyen de classement 3 recevant les données numériques issues de la caméra 1. Le moyen de classement 3 est pourvu d'une mémoire 4 permettant entre autre le stockage desdites données en provenance de la caméra 1. Le moyen de classement 3 est capable de déterminer la phase Anagène, Télogène ou Disparue, dans laquelle est un cheveu présent dans une partie élémentaire de la zone pileuse observée, et le diamètre de chaque cheveu.

En d'autres termes, le moyen de classement 3 reçoit en entrée un fichier image représentatif de la zone pileuse observée et dans lequel chaque lieu élémentaire est affecté d'un niveau de gris ou de caractéristiques de couleur, et émet en sortie un fichier dans lequel chaque cheveu est affecté d'un état, anagène, télogène ou disparu et éventuellement d'autres caractéristiques, notamment concernant les touffes ou bouquets de cheveux.

Le système de simulation comprend un moyen de simulation 5 pourvu d'une mémoire 6 et relié au moyen de classement 3 pour recevoir les données classées comprenant notamment les coordonnées bidimensionnelles d'un follicule pileux, la phase dans laquelle se trouve le cheveu correspondant ainsi que la vitesse de pousse dudit cheveu. Le moyen de classement 3 peut également transmettre au moyen de simulation 5, des données relatives à la densité surfacique de cheveu, à la proportion A de cheveux en phase Anagène, à la proportion T de cheveux en phase Télogène, et à la proportion D de cheveux en phase Disparue.

Le moyen de simulation 5 est capable de fournir en sortie des données de prédiction de l'état individuel de chaque cheveu à un instant futur. En d'autres termes, le moyen de simulation 5 fournit un fichier contenant les coordonnées d'un follicule pileux, la phase dans laquelle va se trouver le cheveu correspondant à une date future, et la date pour laquelle la simulation a été réalisée.

Le système comprend en outre un moyen de généralisation 7 de la simulation à l'ensemble de la chevelure d'un utilisateur. A partir des données de simulation en provenance du moyen de simulation 5, dont les données ne concernent en général qu'une petite surface, par exemple de l'ordre de 1 cm², le moyen de généralisation 7 est apte à fournir en sortie des données semblables à celles fournies par le moyen de simulation 5 mais couvrant une étendue plus grande souhaitée par l'utilisateur, par exemple l'ensemble du cuir chevelu. Le moyen de généralisation 7 reçoit également des données en provenance du moyen de classement 3 dans la mesure où la caméra 1 va effectuer dans le but de la généralisation, la prise d'au moins une image générale de la surface pour laquelle on souhaite effectuer la généralisation, en particulier le cuir chevelu, afin de pouvoir étendre les résultats de la simulation à cette surface plus grande.

Un moyen de visualisation 8 tel qu'un moniteur 9, est relié au moyen de généralisation 7 pour que l'utilisateur puisse voir les résultats de la simulation qui vient d'être effectuée. Une imprimante, non représentée, pourrait également être prévue et reliée au moyen de généralisation 7.

L'évolution possible des phases d'un cheveu est la suivante : un cheveu donné qui se trouve en phase Anagène, c'est-à-dire de croissance, peut, au cours d'une durée élémentaire donnée, rester dans cette même phase ou évoluer vers une phase Télogène ou d'arrêt de croissance. Le passage des cheveux de la phase Anagène à la phase Télogène est un phénomène que l'on peut analyser de façon statistique. Les cheveux restent dans la phase Télogène puis évoluent normalement vers la phase Disparue. Ensuite, les cheveux évoluent normalement, soit vers une nouvelle phase Anagène, soit vers une phase de mort dans laquelle ils restent de façon définitive.

On entend ici par "cycle", le passage d'un cheveu par les trois phases Anagène,Télogène et Disparue, avec retour à la phase Anagène. Toutefois, si l'une de ces trois phases est de courte durée, elle peut être difficilement observable. Le moyen de simulation en tient compte en prévoyant de possibles passages directs A → D, T → A ou encore D → T.

On associe à chaque cheveu un certain nombre de variables permettant de le repérer et de modéliser son évolution, notamment : numéro du cheveu, numéro de la touffe à laquelle appartient le cheveu, diamètre du cheveu, nombre de cycles déjà effectués. A partir de ces données relevées sur une zone élémentaire de surface connue, on calcule la densité de cheveux, le taux de cheveux en phase Anagène, la richesse des touffes ou le nombre moyen de cheveux par touffes, etc. Les cheveux en phases Anagène et Télogène sont classés par diamètre.

On peut ainsi prévoir cinq classes de diamètre pour la phase Anagène et cinq classes de diamètre pour la phase Télogène, la phase Disparue pouvant être assimilée à un diamètre nul.

La modélisation de l'évolution d'un cheveu au cours du temps passe par l'établissement de la loi de la durée de chacune des phases du cycle, et en particulier de la durée $T_A$ de la phase Anagène, mais aussi des matrices de transition entre les onze états du cheveu. On note respectivement $T_T$ et $T_D$, les durées des phases Télogène et Disparue.

La loi de la durée de la phase Anagène est établie tout d'abord par le calcul de trois paramètres a, b, c relatifs à l'alopécie du sujet :

-   a = 1 si la densité de cheveux au cm² est supérieure ou égale à 270, sinon a = 0;

**4**

- b = 1 si le taux d'Anagènes, rapport du nombre de cheveux anagènes à celui des cheveux présents, est supérieur ou égal à 0,8, sinon b = 0;
- c = 2 - min (a, b), c = 1 si le sujet est considéré comme normal, et 2 s'il est considéré comme alopécique.

En notant d le diamètre du cheveu, un modèle de Cox pour la loi de la durée de la phase anagène donne un effet des quatre variables a, b, c et d, avec pour loi de base une loi de Weibull. La probabilité que la durée de la phase Anagène soit égale ou supérieure à une valeur t vaut, pour un cheveu de diamètre d, sur un site de paramètres d'alopécie a, b et c :

$$P(T_A \geq t \mid a, b, c, d) = \exp(-(\lambda t)^{\alpha})$$

avec

$$\alpha = 1 - 1/(5-c)$$

et

$$\lambda = \exp(2-(a+b)/2-0,7*d - |d-3| *0,3)$$

Le coefficient négatif relatif au diamètre indique un allongement de la phase Anagène pour les cheveux de fort diamètre, et quantifie l'impact d'une augmentation de diamètre sur la décélération de la phase anagène du cycle.

Les phases Télogène et Disparue ont un comportement beaucoup moins différencié entre les sites normaux et les sites alopéciques.

La durée de la phase Télogène T peut être modélisée par une loi de Weibull de paramètre $\alpha$ = 1,8 et $\lambda$ = exp (-1,4+0,2*a), et celle de la phase disparue par une exponentielle ($\alpha$ n'est pas significativement différent de 1) de paramètre $\lambda$ = exp(-1+0,2*a).

Les matrices de transition de phase regroupent les probabilités de passage 'd'un état à un instant t à un état à un instant t+1, conditionnellement au fait qu'un changement de phase a eu lieu entre les instants t et t+1.

Trois matrices sont utilisées selon que le cheveu sort de la phase Anagène, Télogène ou Disparue. Ces matrices ont les valeurs suivantes, compte tenu d'un intervalle de confiance d'environ 2%:

| Matrice de transition à partir de la phase Anagène pour les sujets normaux | | | | | | |
|---|---|---|---|---|---|---|
| | D | T1 | T2 | T3 | T4 | T5 |
| A1 | 0,27 | 0,71 | 0,02 | 0,00 | 0,00 | 0,00 |
| A2 | 0,10 | 0,10 | 0,75 | 0,05 | 0,00 | 0,00 |
| A3 | 0,04 | 0,00 | 0,14 | 0,79 | 0,03 | 0,00 |
| A4 | 0,04 | 0,00 | 0,07 | 0,36 | 0,53 | 0,00 |
| A5 | 0,08 | 0,00 | 0,00 | 0,08 | 0,50 | 0,34 |

| Matrice de transition à partir de la phase Anagène pour les sujets alopéciques | | | | | | |
|---|---|---|---|---|---|---|
| | D | T1 | T2 | T3 | T4 | T5 |
| A1 | 0,17 | 0,79 | 0,04 | 0,00 | 0,00 | 0,00 |
| A2 | 0,06 | 0,09 | 0,76 | 0,09 | 0,00 | 0,00 |
| A3 | 0,05 | 0,00 | 0,15 | 0,75 | 0,05 | 0,00 |
| A4 | 0,03 | 0,00 | 0,03 | 0,24 | 0,62 | 0,08 |
| A5 | 0,03 | 0,00 | 0,00 | 0,09 | 0,29 | 0,59 |

| Matrice de transition à partir de l'étape Télogène pour les sujets normaux | | | | | | |
|---|---|---|---|---|---|---|
| | D | A1 | A2 | A3 | A4 | A5 |
| T1 | 0,91 | 0,07 | 0,02 | 0,00 | 0,00 | 0 |
| T2 | 0,86 | 0,03 | 0,08 | 0,03 | 0,00 | 0 |
| T3 | 0,83 | 0,02 | 0,03 | 0,12 | 0,00 | 0 |
| T4 | 0,85 | 0,00 | 0,00 | 0,06 | 0,09 | 0 |
| T5 | 0,60 | 0,00 | 0,00 | 0,20 | 0,20 | 0 |

| Matrice de transition à partir de l'état Télogène pour les sujets alopéciques | | | | | | |
|---|---|---|---|---|---|---|
| | D | A1 | A2 | A3 | A4 | A5 |
| T1 | 0,78 | 0,19 | 0,03 | 0,00 | 0,00 | 0,00 |
| T2 | 0,72 | 0,08 | 0,17 | 0,03 | 0,00 | 0,00 |
| T3 | 0,73 | 0,06 | 0,08 | 0,11 | 0,02 | 0,00 |
| T4 | 0,70 | 0,06 | 0,07 | 0,06 | 0,11 | 0,00 |
| T5 | 0,78 | 0,05 | 0,03 | 0,02 | 0,03 | 0,09 |

| Matrice de transition à partir de l'état Disparus pour les sujets normaux | | | | | | | | | |
|---|---|---|---|---|---|---|---|---|---|
| | A1 | A2 | A3 | A4 | A5 | T1 | T2 | T3 | T4 | T5 |
| D1 | 0,8 | 0,1 | 0,03 | 0,02 | 0,00 | 0,05 | 0,00 | 0,00 | 0,00 | 0,00 |
| D2 | 0,2 | 0,5 | 0,24 | 0,04 | 0,00 | 0,00 | 0,00 | 0,00 | 0,00 | 0,00 |
| D3 | 0,1 | 0,2 | 0,62 | 0,08 | 0,00 | 0,00 | 0,00 | 0,00 | 0,00 | 0,00 |
| D4 | 0,2 | 0,1 | 0,31 | 0,37 | 0,02 | 0,00 | 0,00 | 0,00 | 0,00 | 0,00 |
| D5 | 0,0 | 0,0 | 0,67 | 0,33 | 0,00 | 0,00 | 0,00 | 0,00 | 0,00 | 0,00 |

| Matrice de transition à partir de l'état Disparus pour les sujets alopéciques | | | | | | | | | |
|---|---|---|---|---|---|---|---|---|---|---|
| | A1 | A2 | A3 | A4 | A5 | T1 | T2 | T3 | T4 | T5 |
| D1 | 0,77 | 0,1 | 0,02 | 0,00 | 0,00 | 0,11 | 0,00 | 0,00 | 0,00 | 0,00 |
| D2 | 0,27 | 0,59 | 0,10 | 0,02 | 0,00 | 0,00 | 0,02 | 0,00 | 0,00 | 0,00 |
| D3 | 0,16 | 0,3 | 0,46 | 0,06 | 0,02 | 0,00 | 0,00 | 0,00 | 0,00 | 0,00 |
| D4 | 0,13 | 0,2 | 0,24 | 0,34 | 0,09 | 0,00 | 0,00 | 0,00 | 0,00 | 0,00 |
| D5 | 0,06 | 0,1 | 0,21 | 0,15 | 0,48 | 0,00 | 0,00 | 0,00 | 0,00 | 0,00 |

On remarquera qu'aux cheveux disparus, on affecte un diamètre fictif. Il s'agit en fait du diamètre qui était le leur juste avant leur disparition.

La comparaison des deux dernières matrices montre une plus grande propension au passage "direct" de l'état Disparu à l'état Télogène, sans observation d'un passage par l'état Anagène, des cheveux des sites alopéciques, cela arrive par exemple à environ 10% des cheveux très fins de diamètre I, contre environ 5% chez les sujets normaux.

On peut également tenir compte dans le modèle du nombre de cycles complets réalisés par chaque cheveu. Si un cheveu a atteint un nombre critique de cycles $N_k$, il meurt et ne repousse jamais. $N_k$ est, en général, compris entre 20 et 25, voire 27. On peut donc modéliser un raccourcissement progressif de la durée des phases Anagènes à partir du nombre de cycles déjà effectués par les cheveux.

A partir d'une observation unique, ou même de deux observations espacées, d'un mois par exemple, on n'observe pas les cheveux à l'état disparu. On met donc en oeuvre une estimation du nombre de cheveux disparus qui sont estimés avec leur diamètre avant disparition et la proportion des diamètres des cheveux présents, grâce au moyen de classement 3.

Pour les sujets normaux, on peut tenir compte d'un taux moyen de disparus d'environ 20% et, pour les sujets alopéciques, d'un taux moyen de disparus d'environ 35%. On crée donc ainsi des cheveux fictifs, qui permettent de tenir compte du faible nombre et/ou de la faible durée des observations.

Pour parfaire encore la modélisation, on peut calculer un indice représentatif du taux de couverture du cuir chevelu par les cheveux, en d'autres termes de l'impression visuelle donnée par la chevelure. Cet indice est calculé en additionnant le produit pour chacun des états (A1, A2, A3, A4, A5, T1, T2, T3, T4, T5), du nombre de cheveux dans l'état (phase, diamètre), du diamètre des cheveux et de la longueur de pousse ou de la vitesse de pousse des cheveux.

Dans certains cas et selon le type de coiffure du sujet, on pourrait envisager de tenir compte du carré du diamètre plutôt que du diamètre, par exemple pour une coiffure de type en brosse.

Pour un sujet donné et souhaitant une modélisation de l'évolution de sa chevelure, on effectuera un trichogramme, ou deux trichogrammes espacés d'une durée déterminée, par exemple de l'ordre d'un mois. Chaque trichogramme est effectué sur une surface de l'ordre du cm$^2$. On rase complètement ladite surface d'une zone pileuse, puis quelques jours après, par exemple deux ou trois, on effectue une prise de vue de ladite surface ayant été rasée, au moyen de la caméra 1. On peut ainsi, grâce au moyen de classement, déterminer dans quelle phase se trouve chaque cheveu. En effet, les cheveux en phase Anagène auront nettement poussé, les cheveux en phase Télogène n'auront pas poussé ou quasiment pas poussé, et les cheveux en phase disparue ne seront pas observés.

Dans le cas de deux trichogrammes, le second est effectué de la même façon que le premier, au même endroit du crâne du sujet.

Grâce à l'invention, l'utilisateur bénéficie d'une prédiction à différents temps, de quelques mois à quelques dizaines d'années et qui peut tenir compte d'évolutions rapides du cheveu non observables sur les données ayant servi à établir le modèle. Cette prédiction peut également porter sur des paramètres indirects, tels que le volume ou la couverture de la chevelure en plus des paramètres de phase.

**Revendications**

1. Système de simulation et d'analyse prévisionnelle de l'évolution d'une zone pileuse du derme d'un sujet au cours du temps, caractérisé par le fait qu'il comprend un moyen d'observation de ladite zone pileuse apte à fournir en sortie des données numériques d'observation, un premier moyen de traitement de données numériques capable d'effectuer à partir des données d'observation et de données externes un classement des cheveux de ladite zone, un second moyen de traitement de données numériques capable d'effectuer une simulation de l'évolution des cheveux en fonction des données issues du premier moyen de traitement de données numériques, et un moyen de visualisation des données issues du second moyen de traitement de données numériques, les données en sortie du premier moyen de traitement de données numériques comprenant au moins un classement suivant le diamètre des cheveux.

2. Système selon la revendication 1, caractérisé par le fait que les données en sortie du premier moyen de traitement comprennent en outre un classement des cheveux suivant leur appartenance aux phases Télogène, Anagène ou Disparue.

3. Système selon la revendication 1 ou 2, caractérisé par le fait que les données externes comprennent au moins l'âge du sujet.

4. Système selon l'une quelconque des revendications précédentes, caractérisé par le fait que le second moyen de traitement est capable de calculer la proportion A de cheveux en phase Anagène.

5. Système selon l'une quelconque des revendications précédentes, caractérisé par le fait que le second moyen de traitement est capable de calculer et de prédire la densité surfacique de cheveux, la proportion T de cheveux en phase Télogène, la proportion D de cheveux Disparus, et la vitesse de pousse individuelle des cheveux.

6. Système selon l'une quelconque des revendications précédentes, caractérisé par le fait qu'il comprend un moyen pour effectuer un troisième traitement de simulation de l'évolution de l'ensemble de la chevelure du sujet à partir des données issues du second traitement.

7. Système selon la revendication 6, caractérisé par le fait qu'il comprend un moyen pour associer aux données issues du troisième traitement, des données de simulation de l'évolution du visage.

8. Procédé de simulation et d'analyse prévisionnelle de l'évolution d'une zone pileuse du cuir chevelu d'un sujet au cours du temps, dans lequel :

- on observe ladite zone pileuse pour fournir des données numériques d'observation,
- on effectue un premier traitement numérique des données d'observation pour classer des cheveux de ladite zone pileuse à partir des données d'observation et de données externes,
- on effectue un second traitement numérique pour effectuer une simulation de l'évolution temporelle desdits cheveux en fonction des données issues du premier traitement numérique, et
- on visualise des données issues du second traitement numérique, les données en sortie du premier moyen de traitement comprenant au moins un classement suivant le diamètre des cheveux.

9. Procédé selon la revendication 8, dans lequel on effectue au moins une observation, chaque observation étant précédée par une étape de rasage de ladite zone pileuse, l'étape de rasage étant séparée de l'observation correspondante par une première durée donnée.

10. Procédé selon la revendication 9, dans lequel on effectue au moins deux observations séparées par une deuxième durée donnée.

11. Procédé selon l'une quelconque des revendications 8 à 10, dans lequel à partir des données d'observation, on calcule la couverture capillaire en fonction du nombre, du diamètre et de la longueur ou de la vitesse de pousse des cheveux.

12. Procédé selon l'une quelconque des revendications 8 à 11, dans lequel on effectue un troisième traitement pour effectuer une simulation de l'évolution de l'ensemble de la chevelure du sujet à partir des données issues du second traitement et on visualise les données issues du troisième traitement.

# EP 1 111 535 A1

**Office européen des brevets**

## RAPPORT DE RECHERCHE EUROPEENNE

Numéro de la demande

EP 00 40 3459

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A | LIPOSCAK Z ET AL: "Face recognition from profiles using morphological operations" PROCEEDINGS INTERNATIONAL WORKSHOP ON RECOGNITION, ANALYSIS, AND TRACKING OF FACES AND GESTURES IN REAL-TIME SYSTEMS. IN CONJUNCTION WITH ICCV'99 (CAT. NO.PR00378), CORFU, GREECE 26-27 SEPT 1999, pages 47-52, XP002152958 1999, Los Alamitos, CA, USA, IEEE Comput. Soc, USA ISBN: 0-7695-0378-0 * page 47, colonne de gauche, ligne 1 - page 50, colonne de gauche, ligne 4; figures 1,2 * | 1,8 | G06F19/00 |
| A | DALDEGAN A ET AL: "Creating virtual fur and hair styles for synthetic actors" COMMUNICATING WITH VIRTUAL WORLDS, PROCEEDINGS OF COMPUTER GRAPHICS INTERNATIONAL '93, LAUSANNE, SWITZERLAND, 21-25 JUNE 1993, pages 358-370, XP000961750 1993, Tokyo, Japan, Springer-Verlag, Japan ISBN: 4-431-70125-7 * le document en entier * | 1,8 | |
| A | US 5 764 233 A (BRINSMEAD DUNCAN R ET AL) 9 juin 1998 (1998-06-09) * colonne 2, ligne 33 - ligne 64 * | 1,8 | DOMAINES TECHNIQUES RECHERCHES (Int.Cl.7) G06F G06G G06T |
| A | EP 0 725 364 A (MATSUSHITA ELECTRIC IND CO LTD) 7 août 1996 (1996-08-07) * page 2, ligne 25 - page 4, ligne 8 * | 1,8 | |
| A | EP 0 092 075 A (FRIESEN DORITA) 26 octobre 1983 (1983-10-26) * abrégé * | 1,8 | |

Le présent rapport a été établi pour toutes les revendications

| Lieu de la recherche | Date d'achèvement de la recherche | Examinateur |
|---|---|---|
| LA HAYE | 22 janvier 2001 | Schenkels, P |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

## ANNEXE AU RAPPORT DE RECHERCHE EUROPEENNE
## RELATIF A LA DEMANDE DE BREVET EUROPEEN NO.

EP 00 40 3459

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

22-01-2001

| Document brevet cité au rapport de recherche | | Date de publication | Membre(s) de la famille de brevet(s) | | Date de publication |
|---|---|---|---|---|---|
| US 5764233 | A | 09-06-1998 | DE | 19654807 A | 03-07-1997 |
| | | | GB | 2308961 A,B | 09-07-1997 |
| | | | JP | 9326042 A | 16-12-1997 |
| EP 0725364 | A | 07-08-1996 | JP | 8329278 A | 13-12-1996 |
| | | | US | 6141431 A | 31-10-2000 |
| EP 0092075 | A | 26-10-1983 | DE | 3213806 A | 20-10-1983 |

EPO FORM P0460

Pour tout renseignement concernant cette annexe : voir Journal Officiel de l'Office européen des brevets, No.12/82